Europäisches Patentamt

European Patent Office

Office européen des brevets

(11) Publication number: **0 350 553**
**A1**

(12) **EUROPEAN PATENT APPLICATION**

(21) Application number: 88306438.8

(22) Date of filing: 14.07.88

(51) Int. Cl.⁴: **A61K 9/52 , A01N 25/26**

(43) Date of publication of application:
**17.01.90 Bulletin 90/03**

(84) Designated Contracting States:
**DE FR GB IT**

(71) Applicant: TOPPAN MOORE COMPANY, LTD.
6, Kandasurugadai 1-chome
Chiyoda-ku, Tokyo 101(JP)

(72) Inventor: Utaka, Keiichi
183, Suzuki-cho 1-chome
Kodaira-shi Tokyo-to(JP)
Inventor: Yamada, Hiroshi
16-38 Midori-cho 5-chome
Koganei-shi Tokyo-to(JP)
Inventor: Ono, Kumiko
28-12, Musashidai 3-chome
Fuchu-shi Tokyo-to(JP)

(74) Representative: Allard, Susan Joyce et al
BOULT, WADE & TENNANT 27 Furnival Street
London EC4A 1PQ(GB)

(54) **Sustained-release microcapsules.**

(57) The microcapsule product of the invention has a sustained releasability to emit the core material, which is a hydrophobic and vaporizable liquid such as an organic solvent, perfume or flavour, over months. The capsule walls are made of a condensation product of an amino compound, e.g. melamine, guanamine or urea, and formaldehyde, the molar ratio of formaldehyde to the amino compound to form the capsule walls being within a narrow range of from 1.0 to 1.5. The microcapsule-preparing process is successful only when the weight ratio of the capsule wall material to the core material is in the range of from 3 to 15%

# SUSTAINED-RELEASE MICROCAPSULES

The present invention relates to a novel sustained-release microcapsule product. More particularly, the invention relates to a product comprising microcapsules having specifically designed capsule walls through which a hydrophobic liquid core material having vaporisability and encapsulated therein can be released over a period of time.

The microcapsule product comprises a plurality of particles, each particle having a structure formed of a core material encapsulated in a capsule wall. Various kinds of core materials are widely used in the form of microcapsules including coating materials for pressure-sensitive transfer paper, medicines, agricultural chemicals, perfumes and flavours, adhesives, enzymes, pigments, dyes and solvents.

Several methods are known and practised for the manufacture of microcapsules, including the so-called interfacial polymerization method, in-liquid cure-coating method, in situ polymerization method and coacervation method. The interfacial polymerization method is a method in which a solution of a hydrophobic monomer in a solvent immiscible with water is finely dispersed in water and the polymerization of the monomer is effected at the interface between the aqueous phase and the water-immiscible solvent to form the capsule wall. In the in-liquid cure-coating method, an organic solution of a hydrophobic polymer is introduced in a finely dispersed state into a coagulating bath and cured therein to form capsule walls. In the in situ polymerization method, the core material is introduced into a dispersion medium in finely dispersed state so that the polymerizable monomer contained either in the core material or in the liquid medium is polymerized at the interface between the dispersed phase and the medium to form the capsule walls. In the coacervation method, and aqueous solution of a water-soluble polymer containing the core material finely dispersed therein is admixed with a water-miscible solvent which is a non-solvent for the polymer so that capsule walls of the polymer are formed on the surface of the dispersed phase of the core material.

Various kinds of polymeric materials are used for the formation of the capsule walls, including natural polymers such as gelatin and gum arabic and synthetic polymers such as polyamides, polyurethanes, polyesters, polysulfonamides, polyureas, polysulfonates, polycarbonates, epoxy resins, urea-formal-dehyde resins and melamine-formaldehyde resins.

Together with the rapid expansion in the fields in which microcapsule products have been applied in recent years, the requirements for the performance of microcapsule products are being upgraded year by year. One of these newly upgraded requirements for microcapsule products is directed to the so-called sustained releasability, which means that the core material encapsulated in the capsule walls is gradually emitted through the capsule walls so as to exhibit the desired effect of the core material sustainedly over a length of time.

No particular method is known in the prior art for imparting well controlled sustained-releasability to the capsule walls of microcapsule products without causing any decrease in the mechanical strengths of the capsule walls. One of the methods hitherto proposed was to prepare a blend of several types of microcapsule products in which the microcapsules of each type have capsule walls of different wall thickness or different solubility behaviour, from the other types of microcapsules. This method, however, is not industrially practical in respect of the intensive labour and costs for the preparation of such a blend.

We have now developed a microcapsule product comprising a plurality of microcapsules each having capsule walls imparted with sustained releasability without causing any decrease in the mechanical strength thereof, the core material encapsulated therein can be emitted gradually through the walls to exhibit the desired effect of the core material sustainedly over a period of time and capable of being manufactured industrially with reasonable labour and costs.

Accordingly the present invention provides a microcapsule product composed of a plurality of particles of which each particle comprises a core material, which is a hydrophobic and vaporizable liquid substance, in the form of a droplet; and a capsule wall encapsulating the droplet of the core material, which wall is formed of a condensation product of formaldehyde and an amino compound which is a melamine compound, guanamine compound or urea compound.

As described above, the sustained-release microcapsule product of the invention is characterised by the unique polymeric material of which the capsule walls are formed. Namely, the polymeric material forming the capsule walls of the microcapsule product of the invention is a resin produced by the condensation reaction of formaldehyde and an amino compound which is a melamine compound, guanamine compound or a urea compound.

The above-mentioned three types of amino compounds include not only melamine, guanamine and urea per se, but also various derivatives thereof such as N-methylolated melamines as derivatives of melamine, formguanamine, acetoguanamine and benzoguanamine as derivatives of guanamine and N-methylolated ureas as derivatives of urea. Optionally the formaldehyde may be subjected to the condensation reaction with a combination of the above mentioned amino compound and a phenolic compound such as phenol, o-, m- and p-cresols, or resorcinol. The formaldehyde used in the condensation reaction may conveniently be in the form of an aqueous solution, i.e. formalin, though not particularly limited thereto.

With regard to the molar ratio of the above-mentioned amino compound and formaldehyde in the reaction mixture for the condensation reaction, it is preferred that the molar amount of formaldehyde is in the range of from 1.0 to 1.5 times of the amount of the amino compound assuming that a phenolic compound or another compound having reactivity with formaldehyde is not used in combination therewith.

The above-mentioned molar ratio is unique since it is usual for 3 to 4 moles of formaldehyde to be reacted with a mole of melamine for the preparation of melamine-formaldehyde resins, and 2 to 3 moles of formaldehyde to be reacted with a mole of guanamine or urea for the preparation of guanamine-formaldehyde or urea-formaldehyde resins. The desired sustained-releasability of the capsule walls can be obtained without loss of mechanical strength only when the molar ratio of the reactants is within the above-mentioned range. When the amount of formaldehyde is too small, a considerable portion of the amino compound remains unmethylolated, so that the precondensate may have only an insufficient solubility in water or the capsule walls produced thereby may have a somewhat decreased mechanical strength. When the molar ratio of formaldehyde is too large, on the other hand, the capsule walls produced thereby can no longer exhibit the desired sustained releasability. The exact molar ratio of formaldehyde and amino compound within the 1.0 to 1.5 range and the particular type of the amino compound of course depends on the desired sustained releasability of the capsule walls.

The core material encapsulated in the capsule walls of the above described polymeric material in the inventive microcapsule product is a hydrophobic liquid substance which is vaporizable. Examples of the core material include insecticides and insect-proofing agents, germicides, deodorants, perfumes and flavours, medicines, agricultural chemicals, fertilizers and the like, though not particularly limited thereto provided that the substance is a hydrophobic and vaporizable liquid.

The method for the preparation of the microcapsules of the invention is not particularly limited and any of the conventional methods can be used, including the interfacial polymerization method, in-liquid cure-coating method, in situ polymerization method and coacervation method, of which the in situ polymerization method is particularly preferred. The medium in which the above-mentioned core material, i.e. a hydrophobic and vaporizable liquid substance, is finely dispersed is preferably water or an aqueous mixture mainly composed of water.

The following is a description of a typical process for the preparation of the microcapsule product of the invention. In the first place, the hydrophobic and vaporizable liquid substance as the core material is added to an aqueous acidic medium having a pH of from 3 to 6 and containing a small amount of a copolymer of styrene and maleic anhydride or the like as a dispersing aid. The core material is dispersed or emulsified therein by vigorously agitating the mixture, if necessary, by applying ultrasonic waves. Separately, formalin, i.e. an aqueous solution of formaldehyde is admixed with a small amount of an alkali so as to have an adjusted pH of 8 to 10. The solution is then admixed with the amino compound as described above in a calculated and weighed amount, followed by heating at a temperature in the range from 50 to 100°C to give an aqueous solution of the methylolated amino compound.

Thereafter, the aqueous solution of the methylolated amino compound is added to the aqueous dispersion or emulsion of the core material and the mixture having a pH of 3 to 7 is agitated for 30 to 120 minutes at a temperature of 40 to 90°C to effect the condensation reaction. If necessary, citric acid or another weak acid may be added to the mixture in order to control the pH of the mixture within the above-mentioned range. In this manner, the reaction product of the condensation reaction forms walls around the particles or droplets of the core material to provide an aqueous dispersion of the microcapsules having capsule walls with sustained releasability.

With regard to the relative amount of the condensation product forming the capsule wals, it is preferred that the amount of the wall-forming material is in the range of from 3 to 15% by weight based on the amount of the core material. When the amount of the wall-forming material is too small, completely walled microcapsules can hardly be obtained. When the amount of the wall-forming material is too large, on the other hand, the resultant microcapsules may have somewhat decreased sustained releasability.

When the microcapsules produced in this manner are maintained as a dispersion in the aqueous medium, the core material is stable and never emitted from the microcapsules. Namely, the sustained releasability of the core material is exhibited only after drying the microcapsules to free them from the aqueous medium beforehand or just prior to use. The method for drying the wet microcapsules is not particularly limited and any of the known methods can be used including spray drying, air drying and the like.

The thus prepared sustained-release microcapsule product of the invention is very advantageous when the effect of the core material is desired sustainedly over a period of time with a relatively small amount of the material since the core material, which is a hydrophobic and vaporizable liquid substance, encapsulated within the capsule walls made of a specific polymeric material is emitted gradually through the capsule walls. In addition, the microcapsule product of the invention can be handled either as an aqueous dispersion or as a pre-dried powder, according to need, so that good workability and versatility can be ensured for the respective applications.

In the following, the sustained-release microcapsule product of the invention is described in more detail by way of examples although the scope of the invention is never limited thereby in any sense.

In the examples described below, the sustained releasability of the microcapsule product was evaluated according to the procedure given below. When the product under testing was in the form of an aqueous dispersion, the dispersion was spread and applied to a 10 cm by I0 cm wide sheet of synthetic paper (Yupo #130, a product by Oji Yuka Synthetic Paper Co.) in an amount of about 0.1 g as the content of the microcapsules and then air-dried. When the product under testing was in the form of a dry powder, about 1 g portion of the powder was taken and evenly spread on a watch glass. The thus prepared test sample of microcapsules was kept standing in an atmosphere at a temperature of 23 $^\circ$ C for 1 to 3 months and the amount of the unvaporized core material was calculated from the weight decrease in the microcapsules. In the following description, the terms of "%" and "parts" always refer to "% by weight" and "parts by weight", respectively.

Example 1 (Preparations No. 1 to No. 5).

(Preparation No. 1)

An aqueous solution having a pH of 4.5 to serve as a dispersion medium was prepared by dissolving a copolymeric resin of styrene and maleic anhydride (Scriptset 520, a product by Monsanto Co.) in a concentration of 5% together with a small amount of sodium hydroxide and 300 parts of the aqueous solution were admixed with 200 parts of cyclohexane as the core material. The core material was emulsified in the aqueous medium by use of an ultrahomogenizer (manufactured by Nippon Seiki Co.) The droplets of the emulsified cyclohexane had an average diameter of about 6 μm.

Separately, a solution was prepared by adding 20 parts of melamine and 16.7 parts of a 37% aqueous solution of formaldehyde to 35 parts of water and adjusting the pH at 9.5 with addition of a small amount of a 20% aqueous solution of sodium hydroxide. The molar ratio of formaldehyde to the amino compound, i.e. melamine, in this solution was 1.3. The solution was heated at 85 $^\circ$ C for 15 minutes to prepare an aqueous solution of a melamine-formaldehyde precondensate, which was added to the aqueous emulsion of the core material followed by agitation for 2 hours at 75 $^\circ$ C so that microcapsules were obtained with the core material encapsulated in the capsule walls made of a melamine-formaldehyde resin in the form of a dispersion in the aqueous medium.

(Preparation No. 2)

An aqueous dispersion of microcapsules was prepared in substantially the same manner as in Preparation No. 1 described above except that the cyclohexane as the core material was replaced with the same amount of a grape flavor (FG 8857, a product by Takasago Koryo Kogyo Co.) and that the molar ratio of formaldehyde to melamine was 1.0 instead of 1.3 by using 12.9 parts of melamine and 8.4 parts of the formalin. The aqueous dispersion was then diluted with addition of water to have a content of the microcapsules of 20% and spray-dried into a powdery microcapsule product by using a spray drier (Model DL 41, manufactured by Yamato Kagaku Co.) operated under conditions of a temperature at the inlet port of 250 $^\circ$ C, nozzle pressure of 1 .5 kg/cm$^2$G and feed rate of the dispersion of 15 g/minute.

(Preparation No. 3)

An aqueous dispersion of microcapsules was prepared in substantially the same manner as in Preparation No. 1 described above using cyclohexane as the core material except that the molar ratio of

4

formaldehyde to melamine was 3.5 instead of 1.3 by increasing the amount of the formalin to 45 parts.

(Preparation No. 4)

An aqueous dispersion of microcapsules was prepared in substantially the same manner as in Preparation No. 1 described above except that the cyclohexane as the core material was replaced with the same amount of an orange flavor (PC 8855, a product by Takasago Koryo Kogyo Co.) and that the molar ratio of formaldehyde to melamine was 2.0 instead of 1.3 by increasing the amount of the formalin to 25.7 parts.

(Preparation No. 5)

An aqueous dispersion of microcapsules was prepared in substantially the same manner as in Preparation No. 1 described above using cyclohexane as the core material except that the molar ratio of formaldehyde to melamine was 0.8 instead of 1.3 by decreasing the amount of the formalin to 10.3 parts.

The thus prepared microcapsule products either in the form of an aqueous dispersion or in the form of a dry powder were tested for the sustained releasability and the results are shown in the table below by the percentages of the unvaporized core material after 1, 2 and 3 months. ·

Example 2 (Preparations No. 6 and No. 7).

(Preparation No. 6)

An aqueous solution having a pH of 4.8 to serve as a dispersing medium was prepared by dissolving a copolymeric resin of styrene and maleic anhydride (Scriptset 520, a product by Monsanto Co.) in a concentration of 5% together with a small amount of sodium hydroxide and 300 parts of the aqueous solution were admixed with 200 parts of toluene as the core material. The core material was emulsified in the aqueous medium by use of an ultrahomogenizer. The droplets of the emulsified toluene had an average diameter of about 10 μm.

Separately, a solution was prepared by adding 20 parts of acetoguanamine and 19.3 parts of a 37% aqueous solution of formaldehyde to 35 parts by weight of water and adjusting the pH at 9.5 with addition of a small amount of a 20% aqueous solution of sodium hydroxide. The molar ratio of formaldehyde to the amino compound, i.e. acetoguanamine, in this solution was 1.5. The solution was heated at 80 °C for 15 minutes to prepare an aqueous solution of an acetoguanamine-formaldehyde precondensate, which was added to the aqueous emulsion of the core material followed by agitation for 2 hours at 75 °C so that microcapsules were obtained with the core material encapsulated in the capsule walls made of an acetoguanamine-formaldehyde resin in the form of a dispersion in the aqueous medium.

(Preparation No. 7)

An aqueous dispersion of microcapsules was prepared in substantially the same manner as in Preparation No. 6 described above using toluene as the core material except that the molar ratio of formaldehyde to acetoguanamine was 2.3 instead of 1.5 by increasing the amount of the formalin to 30 parts.

The thus prepared microcapsule products in the form of an aqueous dispersion were tested for the sustained releasability and the results are shown in the table by the percentages of the unvaporized core maaterial after 1, 2 and 3 months.

Example 3 (Preparations No. 8 to No. 10).

(Preparation No. 8)

5

An aqueous solution having a pH of 3.5 to serve as a dispersing medium was prepared by admixing 100 parts of a 10% aqueous solution of a copolymeric resin of ethylene and maleic anhydride (EMA 31, a product by Monsanto Co.) with 20 parts of urea and 2 parts of resorcinol followed by dilution with 200 parts of water and adjustment of pH with a small amount of a 20% aqueous solution of sodium hydroxide. The aqueous solution was admixed with 200 parts of a low-boiling silicone oil (KF 96L, a product by Shin-Etsu Chemical Co.) as the core material, which was emulsified in the aqueous medium by use of an ultrahomogenizer. The droplets of the emulsified silicone oil had an average diameter of about 5 μm.

The aqueous emulsion was then admixed with 35 parts of a 37% aqueous solution of formaldehyde. The molar ratio of formaldehyde to the amino compound, i.e. urea, in this solution was 1.3. The solution was heated at 55 °C for 2 hours so that microcapsules were obtained with the core material encapsulated in the capsule walls made of a urea-formaldehyde resin in the form of a dispersion in the aqueous medium.

(Preparation No. 9)

An aqueous dispersion of microcapsules was prepared in substantially the same manner as in Preparation No. 8 described above using the low-boiling silicone oil as the core material except that the molar ratio of formaldehyde to urea was 1.9 instead of 1.3 by increasing the amount of the formalin to 50 parts.

(Preparation No. 10)

An aqueous dispersion of microcapsules was prepared in substantially the same manner as in Preparation No. 9 described above except that the low-boiling silicone oil as the core material was replaced with the same amount of n-heptane.

The thus prepared microcapsule products in the form of an aqueous dispersion were tested for the sustained releasability and the results are shown in the table by the percentage of the unvaporized core material after 1, 2 and 3 months.

Example 4 (Preparations No. 11 and No. 12).

(Preparation No. 11)

An aqueous solution having a pH of 4.8 to serve as a dispersion medium was prepared by dissolving a copolymeric resin of styrene and maleic anhydride (Scriptset 520, a product by Monsanto Co.) in a concentration of 5% together with a small amount of sodium hydroxide and 300 parts of the aqueous solution were admixed with 200 parts of cyclohexane as the core material. The core material was emulsified in the aqueous medium by use of an ultrahomogenizer. The droplets of the emulsified cyclohexane had an average diameter of about 10 μm.

Separately, a solution was prepared by adding 12.8 parts of acetoguanamine and 19.2 parts of a 37% aqueous solution of formaldehyde to 35 parts of water and adjusting the pH at 9.5 with addition of a small amount of a 20% aqueous solution of sodium hydroxide. The solution was heated at 80 °C for 15 minutes to prepare an aqueous solution of an acetoguanamine-formaldehyde precondensate, which was added to the aqueous emulsion of the core material followed by agitation for 2 hours at 75 °C so that micro-capsules were obtained with the core material encapsulated in the capsule walls made of an acetoguanamine-formaldehyde resin in the form of a dispersion in the aqueous medium. The weight ratio of the thus formed capsule walls to the core material was 10%.

(Preparation No. 12)

The experimental procedure for the preparation of an aqueous dispersion of microcapsules was substantially the same as in Preparation No. 11 described above except that the amounts of the acetoguanamine and formalin were 2.6 and 3.8 parts, respectively. No microcapsules could be obtained in this case. The weight ratio of the capsule walls to be formed to the core material was 2%.

The microcapsule product in the form of an aqueous dispersion prepared in Preparation No. 11 was tested for the sustained releasability and the results are shown in the table by the percentages of the unvaporized core material after 1, 2 and 3 months.

Example 5 (Preparations No. 13 and No. 14).

The experimental procedure for the preparation of an aqueous dispersion of microcapsules in each of these Preparations was substantially the same as in Example 4 described above except that the acetoguanamine-formaldehyde precondensate was replaced with 20 parts (Preparation No. 13) or 45 parts (Preparation No. 14) of a commercial product of a melamine-formaldehyde precondensate (Milben Resin SM 850, a product by Showa Kobunshi Co.) and that the cyclohexane as the core material was replaced with the same amount of benzyl acetate. The weight ratios of the thus formed capsule walls to the core material were 8% and 18%, respectively.

The thus prepared microcapsule products in the form of an aqueous dispersion were tested for the sustained releasability and the results are shown in the table by the percentages of the unvaporized core material after 1, 2 and 3 months.

Example 6 (Preparations No. 15 to No. 17).

(Preparation No. 15)

An aqueous solution having a pH of 3.5 to serve as a dispersion medium was prepared by admixing 100 parts of a 10% aqueous solution of a copolymeric resin of ethylene and maleic anhydride (EMA 31, a product by Monsanto Co.) with 5 parts of urea and 0.5 part of resorcinol followed by dilution with 200 parts of water and adjustment of pH with a small amount of a 20% aqueous solution of sodium hydroxide. The aqueous solution was admixed with 200 parts of an apple flavor (FG 8860, a product by Takasago Koryo Kogyo Co.) as the core material, which was emulsified in the aqueous medium by use of an ultrahomogenizer. The droplets of the emulsified apple flavor had an average diameter of about 5 μm.

The aqueous emulsion was then admixed with 12.5 parts of a 37% aqueous solution of formaldehyde. The solution was heated at 55 °C for 2 hours so that microcapsules were obtained with the core material encapsulated in the capsule walls made of a urea-resorcinol-formaldehyde resin in the form of a dispersion in the aqueous medium. The weight ratio of the thus formed capsule walls to the core material was 5%.

(Preparations No. 16 and No. 17)

The experimental procedure for the preparation of an aqueous dispersion of microcapsules in each of these Preparations was substantially the same as in Preparation No. 15 described above except that the amounts of the urea, resorcinol and formalin were 20 parts, 2 parts and 50 parts, respectively, in Preparation No. 16 and 2 parts, 0.2 part and 5 parts, respectively, in Preparation No. 17. No aqueous dispersion of microcapsules could be obtained in Preparation No. 17. The weight ratios of the thus formed capsule walls or capsule walls to be formed to the core material were 20% and 2% in Preparations No. 16 and No. 17, respectively.

The thus prepared microcapsule products in the form of an aqueous dispersion were tested for the sustained releasability and the results are shown in the table by the percentages of the unvaporized core material after 1, 2 and 3 months.

Example 7 (Preparation No. 18).

An aqueous solution having a pH of 4.5 to serve as a dispersion medium was prepared by dissolving a copolymeric resin of styrene and maleic anhydride (Scriptset 520, a product by Monsanto Co.) in a concentration of 5% together with a small amount of sodium hydroxide and 300 parts of the aqueous solution were admixed with 200 parts of benzene as the core material. The core material was emulsified in the aqueous medium by use of an ultrahomogenizer. The droplets of the emulsified benzene had an average diameter of about 4 μm.

Separately, a solution was prepared by adding 5 parts of melamine and 4.8 parts of a 37% aqueous solution of formaldehyde to 35 parts of water and adjusting the pH at 9.5 with addition of a small amount of a 20% aqueous solution of sodium hydroxide. The solution was heated at 80 °C for 15 minutes to prepare an aqueous solution of a melamine-formaldehyde precondensate, which was added to the aqueous emulsion of the core material followed by agitation for 2 hours at 75 °C so that microcapsules were obtained with the core material encapsulated in the capsule walls made of a melamine-formaldehyde resin in the form of a dispersion in the aqueous medium. The weight ratio of the thus formed capsule walls to the core material was 3.4%.

The thus prepared microcapsule product in the form of an aqueous dispersion was tested for the sustained releasability and the results are shown in the table by the percentages of the unvaporized core material after 1, 2 and 3 months.

### Table

| Example No. | Preparation No. | Percentage of unvaporized core material, after | | | Sustained releasability |
|---|---|---|---|---|---|
| | | 1 month | 2 months | 3 months | |
| 1 | 1 | 72 | 51 | 23 | Yes |
| | 2 | 78 | 57 | 28 | Yes |
| | 3 | 100 | 100 | 100 | No |
| | 4 | 100 | 100 | 100 | No |
| | 5 | - | - | - | - |
| 2 | 6 | 59 | 38 | 13 | Yes |
| | 7 | 100 | 100 | 100 | No |
| 3 | 8 | 65 | 42 | 29 | Yes |
| | 9 | 100 | 100 | 100 | No |
| | 10 | 100 | 100 | 100 | No |
| 4 | 11 | 68 | 42 | 15 | Yes |
| | 12 | - | - | - | - |
| 5 | 13 | 70 | 53 | 19 | Yes |
| | 14 | 100 | 100 | 100 | No |
| 6 | 15 | 60 | 39 | 16 | Yes |
| | 16 | 100 | 100 | 100 | No |
| | 17 | - | - | - | - |
| 7 | 18 | 52 | 31 | 10 | Yes |

## Claims

1. A microcapsule product composed of a plurality of particles of which each particle comprises a core material, which is a hydrophobic and vaporizable liquid substance, in the form of a droplet; and a capsule wall encapsulating the droplet of the core material, which wall is formed of a condensation product of formaldehyde and an amino compound which is a melamine compound, guanamine compound or urea compound.

2. A microcapsule product as claimed in claim 1 wherein the capsule wall is a condensation product of formaldehyde and the amino compound in a molar ratio in the range of from 1.0 to 1.5.

3. A microcapsule product as claimed in claim 1 or claim 2 wherein the weight of the capsule wall is in the range from 3 to 15% by weight of the weight of the core material.

4. A microcapsule product as claimed in any one of the preceding claims wherein the core material is an insecticide, insect-proofing agent, germicide, deodorant, perfume, flavour, medicine, agricultural chemical or fertilizer.

5. A microcapsule product as claimed in any one of the preceding claims which is in the form either of an aqueous suspension of the microcapsules or a dried powder.

European Patent Office

**EUROPEAN SEARCH REPORT**

Application Number

EP 88 30 6438

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| L | CHEMICAL ABSTRACTS vol. 110, no. 1, 2nd January 1989, page 660, column 1, abstract no. 7081a, Columbus, Ohio, US; K. NAKAYAMA et al.: "Manufacture of urea-formaldehyde slow-release nitrogen fertilizers" & JP - A - 63 129 088 (K. NAKAYAMA et al.), 01-06-1988 (Cat. X) * abstract * | 1-5 | A 61 K 9/52<br>A 01 N 25/26 |
| X | CHEMICAL ABSTRACTS vol. 108, no. 19, 9th May 1988, page 570, column 2, abstract no. 166573n, Columbus, Ohio, US; E. UNO et al.: "Manufacture of slow-release nitrogen fertilizer containing UF condensates" & JP - A - 62 223 084, 01-10-1987 * abstract * | 1,2,4,5 | |
| A | idem | 3 | |
| X | EP-A-0 059 949 (PRINTEX SA) * claims * | 1,3-5 | TECHNICAL FIELDS SEARCHED (Int. Cl.4) |
| A | | 2 | A 61 K<br>A 01 N<br>C 08 G |
| X | US-A-4 157 983 (R. GOLDEN et al.) * column 3, lines 7-9; claims * | 1,2,4,5 | |
| A | | 3 | |
| X | EP-A-0 025 255 (E.J. DU PONT DE NEMOURS & CO) * page 5, lines 31-35; claims * | 1,2,4,5 | |
| A | -/- | 3 | |

The present search report has been drawn up for all claims

| Place of search | Date of completion of the search | Examiner |
|---|---|---|
| BERLIN | 10-02-1989 | SIATOU E |

European Patent
Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-3 629 714  (VEB CHEMIEKOMBINAT BITTERFELD) * page 4, table 1; claims * | 1,2,4,5 | |
| A | DE-A-3 629 714 | 3 | |
| X | PATENT ABSTRACTS OF JAPAN vol. 5, no. 114 (C-64) (786), 23rd July 1981; & JP - A - 56 55310 (S. MITSUBISHI et al.) * abstract * | 1,4,5 | |
| A | idem | 2,3 | |
| X | GB-A-1 304 375  (L'OREAL) * page 3, lines 7-9; claims * | 1,3,4 | |
| A | | 2,5 | |
| X | US-A-3 584 113  (T. TAKEBE et al.) * claims * | 1,4,5 | |
| A | | 2,3 | |
| X | EP-A-0 200 288  (INTERNATIONAL SPIKE INC) * claims * | 1,4,5 | TECHNICAL FIELDS SEARCHED (Int. Cl.4) |
| A | | 2,3 | |
| X | EP-A-0 227 987  (TOPPAN MOORE COMPANY LTD) * claims * | 1,4 | |
| A | -/- | 2,3,5 | |

The present search report has been drawn up for all claims

| Place of search | Date of completion of the search | Examiner |
|---|---|---|
| BERLIN | 10-02-1989 | SIATOU E |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)

European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 591 857  (KUREHA KAGAKU KOGYO KABUSHIKI) <br> * example 1; claims * <br> --- | 1,4,5 | |
| A | FR-A-2 591 857 <br> --- | 2,3 | |
| X | JOURNAL OF APPLIED POLYMER SCIENCE vol. 22, no. 4, May 1978, pages 1015 - 1020, John Wiley Sons Inc., New York, US; A. SIEGMANN et al.: "Controlled release of Propham from Plastic Granules" * whole article * | 1,4,5 | |
| A | idem <br> ----- | 2,3 | |
| | | | TECHNICAL FIELDS SEARCHED (Int. Cl.4) |

The present search report has been drawn up for all claims

| Place of search | Date of completion of the search | Examiner |
|---|---|---|
| BERLIN | 10-02-1989 | SIATOU E |